# NEW EUROPEAN PATENT SPECIFICATION

(11) **EP 0 449 116 B2**
(45) Date of publication and mention of the opposition decision: **25.08.2004**
(45) Mention of the grant of the patent: 11.08.1999
(21) Application number: 91104432.9
(22) Date of filing: 21.03.1991
(51) Int. Cl.: C12N 15/48, C12N 15/49, C12N 15/62, A61K 39/21

(54) **DNA sequences encoding modified retroviral gag polypeptides and vaccines containing them or aggregates thereof**
Für abgeänderte retrovirale GAG-Polypeptide kodierende DNA-Sequenzen und diese enthaltende Impfstoffe oder Aggregate davon
Séquences d'ADN codant pour des polypeptides rétroviraux modifiés et vaccins les contenant ou aggrégats de ceux-ci

(30) Priority: 21.03.1990 EP 90105319
(43) Date of publication of application: 02.10.1991
(73) Proprietor: Geneart GmbH, 93053 Regensburg (DE)
(72) Inventor: Wolf, Hans, Prof. Dr., W-8130 Starnberg (DE); Fliessbach, Holger, Dipl.-Biol., W-8000 München 2 (DE); v. Brunn, Albrecht Dr., W-8000 München 71 (DE); Wagner, Ralf, Dipl.-Biol., W-8000 München 71 (DE); Motz, Manfred, Dr., W-8000 München 70 (DE)
(74) Representative: VOSSIUS & PARTNER

(56) References cited:
- EP-A- 0 343 132
- GB-A- 2 188 639
- ANALYTICAL BIOCHEMISTRY, vol. 161, no. 2, March 1987, pages 370-379; R.L. SHOEMAN et al.: "Comparison of recombinant human immondeficiency virus gag precursor and gag/env fusion proteins and a synthetic env peptide as diagnostic reagents"

## Description

The technical problem underlying the present invention is to provide DNA sequences encoding modified retroviral p55-gag polypeptides that can be used for the immunization of mammals against retroviral diseases. This invention particularly relates to a newly designed recombinant HIV subunit vaccine, produced in appropriate expression systems like e.g. recombinant baculo- or vaccinia viruses in insect cells or mamalian cells, respectively. The invention is based on the HIV-1 group specific antigen p55-gag constituting the viral core and its particle forming capacity, when produced in eukaryotic cells. The immunogenicity of the premature 90-110 nm p55-core particles can be extended by Inserting well characterized epitopes of other HIV-1, HIV-2 or SIV derived reading frames (e.g. env, nef, pol) into 3 different regions of the gag coding sequence that were selected by computer assisted analysis of the p55 secondary structure. To demonstrate the universal principle of stable expression and enhanced immunogenicity of recombinant p55/gp120 particles, sequences examplified by the gp120 CD4-binding region, a consensus sequence of the major gp120 neutralizing epitope V3 and a sequence of gp41 ("fusogenic region") were inserted into the coding sequence of the p55 carrier protein and the resulting constructs expressed by recombinant vaccinia- and baculo viruses. Recombinant p55/gp120 vaccinia viruses can be applied directly as live vaccines or as source of protein antigen. Recombinant baculoviruses are used for efficient production and purification of the particular and chimeric p55/gp120 subunit vaccines only.

With regard to the ability of HIV, to persist latently in infected cells, the elimination of free virus by neutralizing antibodies as well as the clearance of infected cells by antibody dependent cell cytolysis (AD-CC) and cytotoxic T-cells is of decisive significance for desease progression. In order to learn more about how the immune system deals with free virus or HIV infected cells, many labs focused on the identification and characterisation of B- and T-cell epitopes, including all reading frames. The role of humoral immune response is crucial in HIV infection. During all stages of disease progression, exept the final stages of AIDS, antibodies to almost all HIV proteins can be detected (Vornhagen et al., 1989). Neutralizing epitopes directed to the outer structural proteins gp120 (Goudsmit et al., 1988; Palker et al., 1988), gp41 (Chan et al., 1986) and to the inner structural proteins p17 (Papsidero et al., 1989) and p24 (Wolf et al., 1990) were described, that might play a role in the elimination of free virus. Antibody dependent cell mediated cytotoxisity (ADCC; Lyerly et al., 1987,1988) may be important for elimination of HIV infected cells. One such ADCC epitope seems to be located in the C-terminal part of gp120. However, antibodies are also known to influence progression of disease negatively (Fc and/or complement mediated antibody enhancement; Takeda et al., 1989, Homsey et al., 1989, Robinson et al., 1989). The proliferation of T4 cells can be group specific and was proven to be directed towards distinct env, gag and pol epitopes by application of synthetic peptides (Schrier et al., 1989). T-cell mapping with recombinant gp120 vaccinia viruses identified four T-helper epitopes on the gp120 (Berzofsky et al., 1988, Krohn et al., 1988). Moreover, three p24 specific CD4 positive T-cell lines could be established. MHC class I restricted CTL epitopes were defined for env (Takahashi et al., 1988, Siciliano et al., 1988), gag (Nixon et al., 1988) and pol proteins (Walker et al., 1988, 1989). CTL mediated lysis is of outranging importance for the elimination of infected cells. Other virus systems (Influenza-, Rabies-, Cytomegalo virus) indicated, that non structural proteins are capable to generate immunity by induction of cytotoxic T-cells, and that a priming of the immune system is achieved, which upon challenge, causes a more effective response even towards other viral proteins (Milich et al., 1988).

The use of total HIV or of intact HIV encoded genes can not exclude adverse effects upon immunization. Amongst such negative effects are antibody enhancement, autoimmune reactions, induced tolerance or allergic side effects.

Despite initial encouraging in vitro results immunization with complete gp160 or subunits does not seem to be sufficient to induce protective immunity. The neutralizing properties of the induced immune response was restricted to the same isolate, used for immunization (type-, not group specific; Nara et al., 1988). Additionally, it was shown, that HIV can still amplify in gp160 immunized animals after challenge (Hu et al., 1987). Several reasons might explain the failure of protective immunity:
1. The decisive epitopes of gp120 vary faster than the elimination by antibodies.
2. The virus spread is mediated at the beginning of the infection by direct cell-cell contact. A humoral immune response is not sufficient and a cellular response to gp120 eventually not sufficient.
3. Enhancing antibodies may negatively influence disease progression. Complement dependent and Fc-mediated enhancement of HIV infection has been shown in different in vitro studies (Takeda et al., 1988, Homsey et al., 1989, Robinson et al., 1989).
4. Epitopes which mimic products of normal cellular genes elict immune responses, which attack normal cells (Reiher et al., 1986, Golding et al., 1988, 1989).
5. Immunosuppressive effects of the HIV envelope: free gp120 binds with high affinity to the CD4 receptor molecule of target cells and labels them for AD-CC(Lyerly et al., 1988) and CTL attack in vitro (Siliciano et al., 1988, Lancavecchia et al., 1988). Furthermore. there is evidence that binding of gp120 to the CD4 molecule interferes with the region of the CD4 receptor, which is necessaryforthe interaction with MHC class II molecules (Weinhold et al, 1989, Clayton et al., 1989). Moreover, regions of gp 120 homologous to those of the transmembrane glycoprotein of human type C retroviruses known to be highly suppressive for macrophage function are also present in HIV gp41 (Clanciola et al., 1988).

Thus, the technical problem underiying the present invention is to provide DNA sequences encoding a modified retroviral p55-gag polypeptide that can be used for the immunization of mammals against retroviral diseases.

The solution to the above technical problem is achieved by providing the embodiments characterized in the claims.

Accordingly, the present invention relates to a DNA sequence encoding a modified retroviral gag polypeptide as characterized in the claims, said gag polypeptide containing at least one amino acid sequence representing a non-gag polypeptide antigenic determinant, wherein the DNA sequence is derived from the retrovirus HIV-1 wherein the gag polypeptide is p55. wherein said amino acid sequence substitutes a region(s) of said gag polypeptide, said region(s) being capable of presenting the inserted amino acid sequence to the immune system so as to elicit an immune response and wherein the region referred to above is located at amino acid positions 99 to 154, 211 to 241, or 436 to 471 or at any combination thereof.

Such an immune response either is a humoral or a cell-mediated immune response. Such immune responses involve the B-cell and/or T-cell compartment.

The term "of presenting" refers to the availability of an antigen for immunerecognition in a way to initiate primary and /or recall immune response.

The regions into which said amino acid sequences are substituted are selected according to the following criteria:
(a) hydrophilicity,
(b) flexibility and
(c) surface probability of the deleted region.

The above referred to amino acid sequence replaces gag polypeptide sequence positions.

In a preferred embodiment of the present invention the DNA sequence encoding said additional amino acid sequence is inserted via a linker. Such linkers are conventional linkers which may contain several restriction enzyme cleavage sites permitting the incorporation of DNA sequences encoding the above mentioned additional amino acid sequence according to conventional methods.

In preferred embodiments of the present invention the non-gag polypeptide antigenic determinant is:
(a) at least a part of the CD4-binding domain of gp120:
(b) at least a part of the variable region 3 of gp120; or
(c) at least a part of a conserved region of gp 41.

Particularly preferred pans of the CD4-binding domain of gp120 are derived from the V3 region thereof and include the subregions and derivatives of said V3 region which are described in aetail in the European Patent Application claiming priority of EP 90 10 5313.2

The above-mentioned parts of the proteins are selected regions thereof which are capable of inducing an immune response to the naturally occurring virus. Preferably they are capable of inducing the formation of antibodies which react with the naturally occurring virus or a cell-mediated immune response to infections with said virus.

In another embodiment the invention relates to recombinant vectors containing any of the DNA sequences referred to hereinbefore.

Said recombinant vectors include conventional plasmids which may be used for the cloning or for the expression of the DNA sequence in a host cell. In case of expression piasmids the DNA sequence is preferably combined with a promoter sequence controlling its expression in suitable host cells.

In a further embodiment the invention relates to recombinant viruses containing a DNA sequence as referred to hereinbefore. Preferably, this recombinant virus is derived from vaccinia virus.

The invention also relates to host organisms which are transfqrmed by said recombinant vectors or recombinant viruses.

Furthermore, the present invention relates to a modified retroviral gag polypeptide which is encoded by any of the DNA sequences referred to above.

The present invention additionally relates to aggregates which are in the form of particles.

In a further embodiment the present invention relates to a method for the production of a modified retroviral gag polypeptide as characterized hereinbefore said method comprising cultivating a host organism as mentioned above on the suitable conditions and recovering the expression product from the medium.

Its a further specific embodiment of the invention to produce said gag particles in a baculo virus dependent expression system in Spodoptera frugiperda insect cells and, using stably transfected cell lines, in Drosophila Schneider cells.

Finally, the invention relates to a vaccine containing a recombinant virus as described hereinbefore, a polypeptide forming particles as described hereinbefore. In preferred embodiments these vaccines also contain a pharmaceutically acceptable carrier and/or diluent.

To develop a recombinant designed vaccine, that is capable to induce protective immunity to HIV infection, detailed knowledge about antigenicity and variability of structures, that are involved in virus adsorption and penetration are necessary. Few alternative approaches are based on HIV derived gp120 depleted or partially depleted particles or purified gag protein.

### The following rationales are bases of our present invention

1. Because of the observed variability of HIV large parts of immunologically relevant gene products must be included.
2. Epitopes known to elict enhancing antibodies and epitopes known to mimic cellular proteins, should be excluded.
3. Those regions, that induce T-cell immunity and that are targets off ADCC, must be taken into consideration as components of the vaccine.
4. The vaccine product should be generated as a particulate antigen to act as immunogen in a stable form without application of additional adjuvants.
5. Alternatively recombinant viruses such as Vaccinia, Herpes simplex or Adenovirus can be used to express these components directly in infected cells.

The possibility to produce premature p55-core particles in eukaryotic expression systems enables us to design a subunit vaccine, that performs the above mentioned postulates. Autologous p55-core particles are expressed by our group and others in the vaccinia and baculo expression system. As known from other particular subunit vaccines ( e.g. Hep. B 22 nm particles), particulate structures show an intrinsic adjuvant potential which is used in our invention.

### Production of recombinant p55/gp120 hybrid particles according to the invention

As a consequence of all findings, we used p55-core particles as an autologous carrier for appropriate epitopes out of the context of other reading frames.
1) p55-gag was cloned into a comercial pUC-vector and expressed in E. coli strain JM109. For cloning of the authentic p55 protein without untranslated sequences the PCR (Polymerase Chain Reaction) was used. Following expression of p55 in eukaryotic systems was achieved by recombinant Vaccinia and Baculo viruses. The formation of p55 particles was proven by sucrose gradient sedimentation and electron microscopy.
2) The identification of appropriate positions in the p55-core protein for the insertion of epitopes from other reading frames was performed with computer assisted analysis of the p55 secondary structure (Wolf et al., 1988). By this approach we predicted four regions within the p55-core protein being exposed on the surface of the protein.
3) For the developement of the recombinant p55-core/gp120-env sequences a polylinker was synthesised. This linker sequence contained all restriction sites, which were necessary for the development and following expression of three distinct p55 deletion constructs each missing a region coding for 30-45 amino acids. By this compatibility of all deletion constructs for insertion of foreign sequences, for subcloning and following expression in eukaryotic vector systems was achieved.
4) In a first approach, three epitopes from the HIV-1 gp120-env reading frame were selected for insertion in the p55 deletion constructs:

### The CD4 binding region

Adsorption of free virus and HIV infected cells to CD4 target cells is directed by the binding of the viral external membrane protein gp120 to the cellular membrane protein CD4. The CD4 receptor molecule is mainly expressed on T4-lymphocytes and cells of the mono- cyte- and macrophage lineage. The region being responsible for the interaction with the CD4 receptor is located in the C-terminal part of gp120 (aa413-451) and well conserved in different HIV isolates (Kowalski et al., 1987, Lasky et al., 1987). Although the CD4 binding region seems not to be immunogenic in humans in natural infection, it was shown that the insertion of this region into the VP1 surface structural protein of poliovirus induces neutralizingantibodies in infected animals. Moreover, monoclonal antibodies, directed towards this sequence have neutralizing capacity and inhibit syncytia formation in vitro by blocking CD4 dependent attachment of free virus or HIV infected cells to the target cells (Sun et al., 1989).

### GPGR-V3-loop of gp120

The hypervariable V3-loop of the gp120 was described from our group as the only hypervariable region without consensus sequence for N-glycosylation and discussed in its immunological importance (Modrow et al., 1987). This region was identified to be the major HIV-1 neutralizing epitope and could be correlated with a 24 aa sequence (aa 302-326). A 6 amino acid peptide is sufficient to induce neutralizing antibodies. This sequence contains a 4 aa beta turn motiv, GPGR, that is highly conserved in different isolates (Palker et al., 1988, Goudsmit et al., 1988). Flanking amino acids seem to be critical for antibody binding and type specific virus neutralization. A 14 aa sequence (315-329) was characterized to be an ADCC target region. Moreover, it could be shown, that MHC class I restricted cytotoxic T-cells recognize a 18 aa peptide of the V3 region in the mouse model (Takahashi et al., 1988). How anti-V3-neutralizing antibodies actually inhibit infection and syncytia formation in vitro (Skinner et al., 1988) is not clear to date. Possible hints are given by recent results, that indicate a processing of gp120 under certain assay conditions in 2 polypeptides (50 KD, 70 KD). The cleavage site was mapped directly to the conserved sequence GPGRA of the V3-loop. The processed gp120 is still able to bind to the CD4 molecule, but can't be any longer recognized by anti-V3-neutralizing antibodies. Trypstatin, a new Kunitz-type protease inhibitor with a high sequenze homology to the gp120-V3 region, inhibits the described protease activity and the following syncytia formation. The V3 dependent, postbinding processing event seems to be a predisposition for the fusion of the outer viral membrane or the membrane of HIV-1 infected cells with a CD4 target cell (Hattori et al., 1989). This essentially could explain the inhibitory effect of anti-V3-neutralizing antibodies. Recent evidence indicates that the deletion of this loop from an infectious clone abolishes infectivity of the virus.

### The gp41 conserved region

The gp41 mediates the membrane fusion of bound HIV with the CD4 target cell and the syncytia formation of infected with uninfected cells (Kowalski et al., 1987, Mc Clure et al., 1988). Monoclonal antibodies raised to a gp41 group specific antigen neutralize a panel of different HIV-1 isolates in vitro. Insertion of the gp41 neutralizing epitope into the poliovirus VP1 and following immunization of experimental animals resulted in the induction of anti-gp41-neutralizing antibodies, that inhibited syncytia formation (Evans et al., 1989).

The CD4 binding region and the gp41 fusogenic region were amplified and subcloned into the p55 deletion constructs using PCR reaction. The consensus sequence of the major gp120 neutralizing epitope V3 was chemically synthesized and also inserted into the p55 deletion clones. To determine correct expression of the designed p55/gp120 fusion proteins, the recombinant proteins were produced in E. coli and verified by Western blot analysis.
5) After subcloning of the final recombinant p55/gp120 constructs into eukaryotic transfer vectors these hybrid genes were expressed in vaccinia- and baculo virus expression systems. The correct expression was verified by Western blot analysis and immunofluorescence. By electronmicroscopy particle formation was studied.

### Brief description of the figures [0033]

- Fig.1:: Description of the cloning procedure to the E. coli expression clone pUC8p55

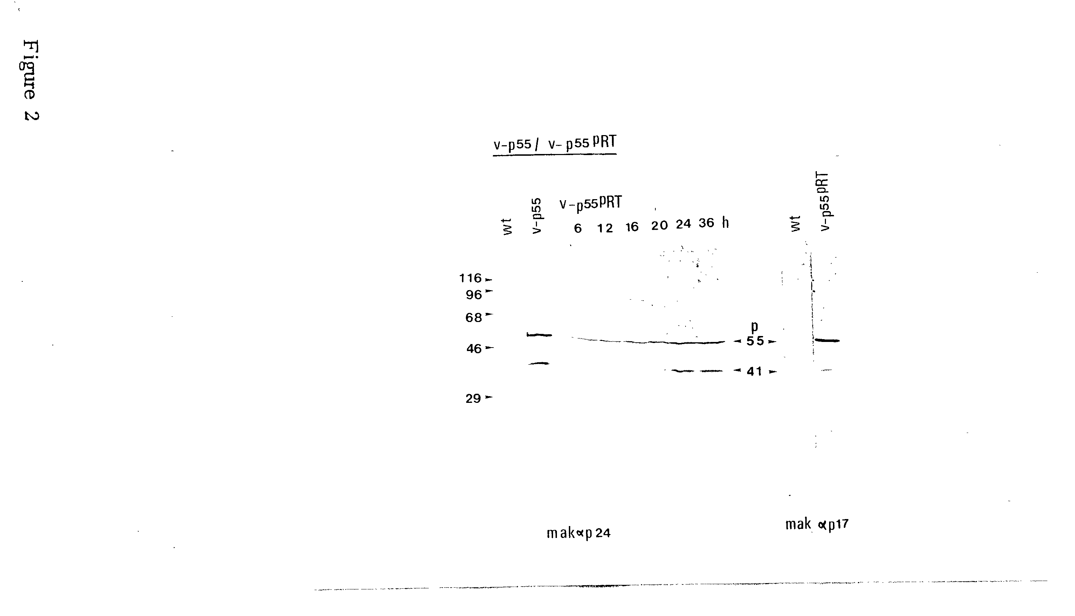

- Fig.2:: Expression of p55 (v-p55) and p55 in combination with the HIV protease (v-p55prt) by recombinant vaccinia viruses. CV1 cells were infected with 10 pfu of p55 recombinant vaccinia virus. The cells were harvested after 36 hours (v-p55) and at different intervals after infection and resuspended in boiling mix. The infected cells were assayed by conventional Western blot analysis as follows: Cell lysates were separated in a 17.5% PAGE and blotted on nylon membranes (Schleicher and Schuell). 55KD and 41KD recombinant proteins were detected by monoclonal antibodies directed to p24 (mab 13/5) and to p17. As proven by the experiment, the HIV-1 protease has no influence on the expression of p55 in the vaccinia expression system. As shown by the reaction of the shortened 41 KD product with the anti-p17 monoclonal antibody, at least part of the shortened product shares the N-terminal sequences with the complete 55KD protein.
- Fig.3:: Expression of p55 by recombinant baculoviruses (b-p55). Spodoptera frugiperda cells were infected with 10 pfu of said virus. Infected cells were harvested daily until day 5. Cells from day 1-5 and culture supernatant from day 5 were resuspended in boiling mix and assayed by conventional Western blot analysis. Recombinant proteins were specifically detected by monoclonal antibodies directed to p24.
- Fig.4:: 2ml of culture supernatant of b-p55 infected cells, harvested 5 days after infection, were assayed by sucrose density sedimentation (2,5 hours, TST 41.14; 28000 rpm). 19 fractions (600 µl) were collected and 20 µl of each fraction were analyzed by conventional Western blot analysis using p24 specific monoclonal antibodies for the detection of the recombinant protein. As proven by following negativ staining, premature p55 particles accumulated in fraction 17 at a sucrose density of 45%.
- Fig.5:: Spodoptera frugiperda cells, infected with 10 pfu b-p55, were fixed 5 days after infection in 2% glutaraldehyd and assayed for particle formation by ultrathin section electron microscopy. Premature 90-110 nm p55 particles were detected budding from the cytoplasmic membrane.
- Fig.6:: 4 regions were predicted by computer assisted analysis of the p55 secondary structure to be exposed epitopes on the surface of the core protein. Those regions were deleted and supplemented by small polylinkers, selected for simple integration of foreign epitopes.
- Fig.7:: synthetic primer sequences used in PCR reaction, small synthetic linker fragments to make the p55 deletion constructs compatible to each other with regard to the reading frame for the insertion of foreign epitopes and a synthetically produced epitope of the HIV-1 gp120.
a) synthetic polylinker for the construction of the pUC8 derived plin8-vector.
b) synthetic primers used for amplification of the 3 part of the p55 deletion construct-4.
c) synthetic primer sequence necessary for amplification of the HIV-1 derived CD4 binding region of gp120.
d) synthetic primer sequence used for amplification of the HIV-1 derived gp41 fusogenic region.
   e) sequence of a chemically synthesized consensus region of the major HIV-1 neutralizing epitope V3.

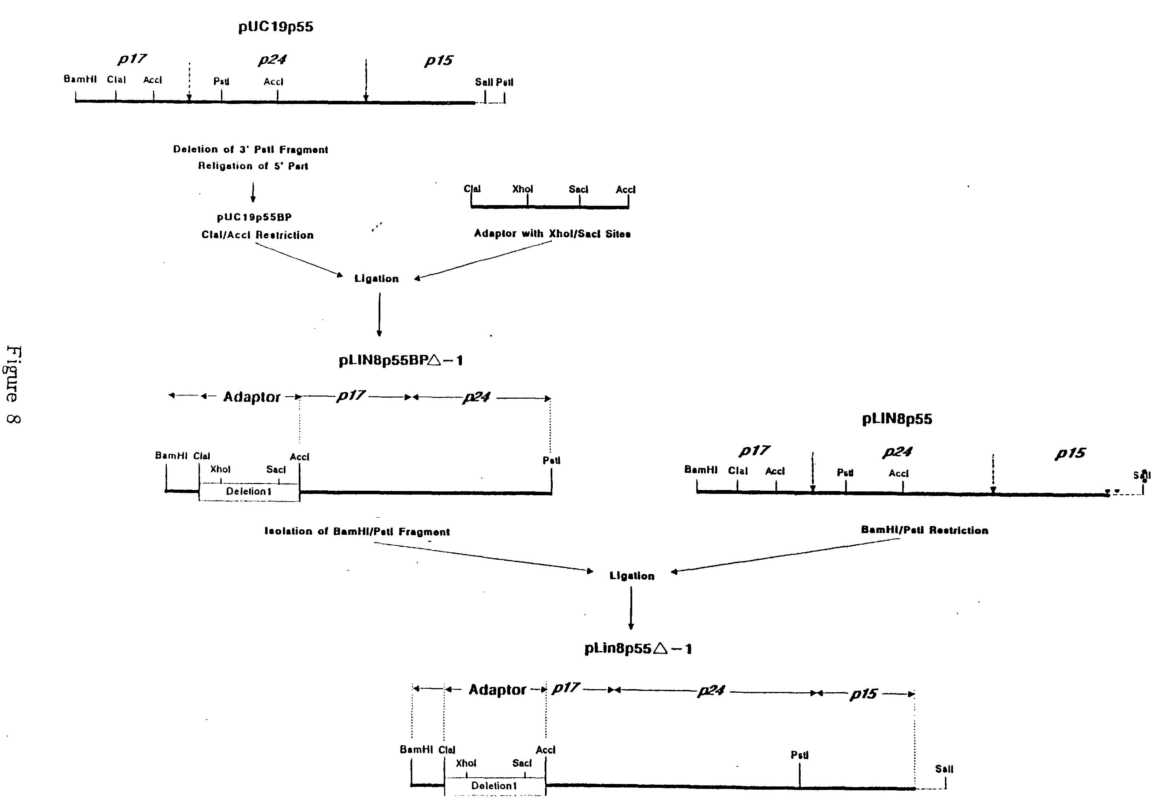

- Fig.8:: cloning procedure for development of the plin8p55 1 construct.

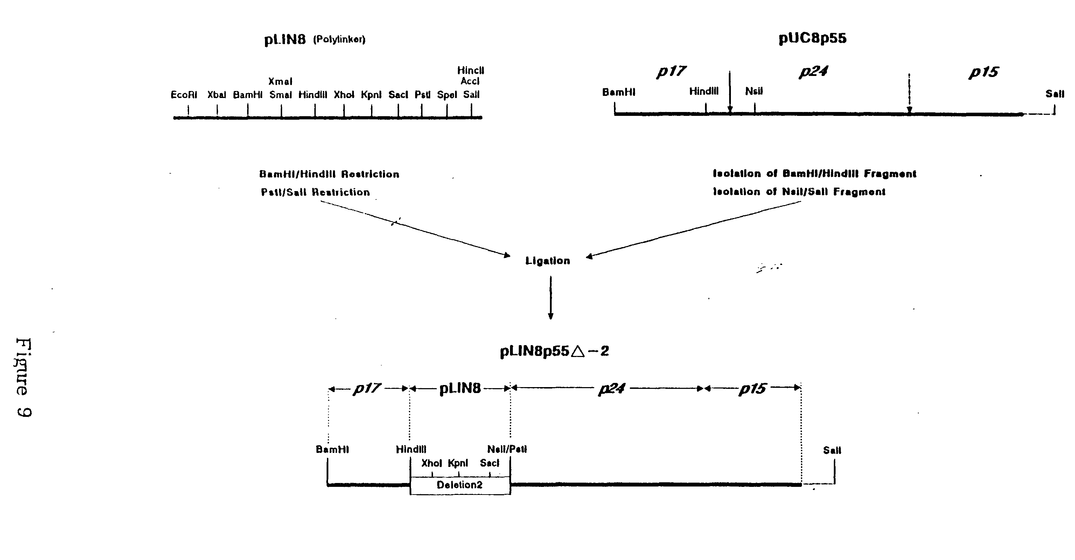

- Fig.9:: cloning procedure for development of the plin8p55 2 construct.

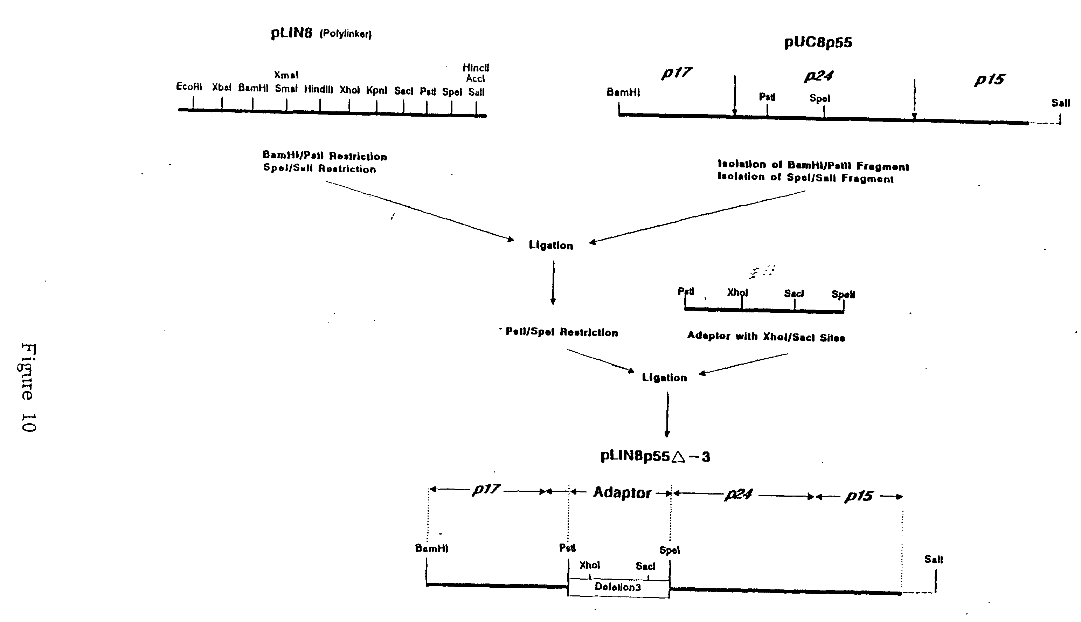

- Fig.10:: cloning procedure for development of the plin8p55 3 construct.

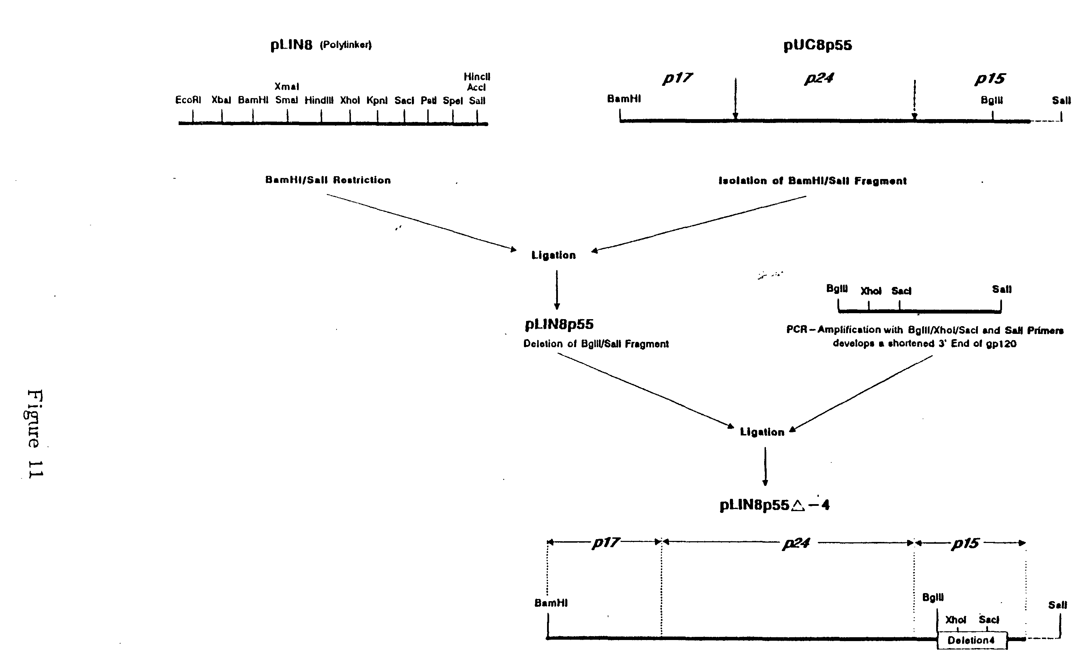

- Fig.11:: cloning procedure for development of the plin8p55 4 construct.

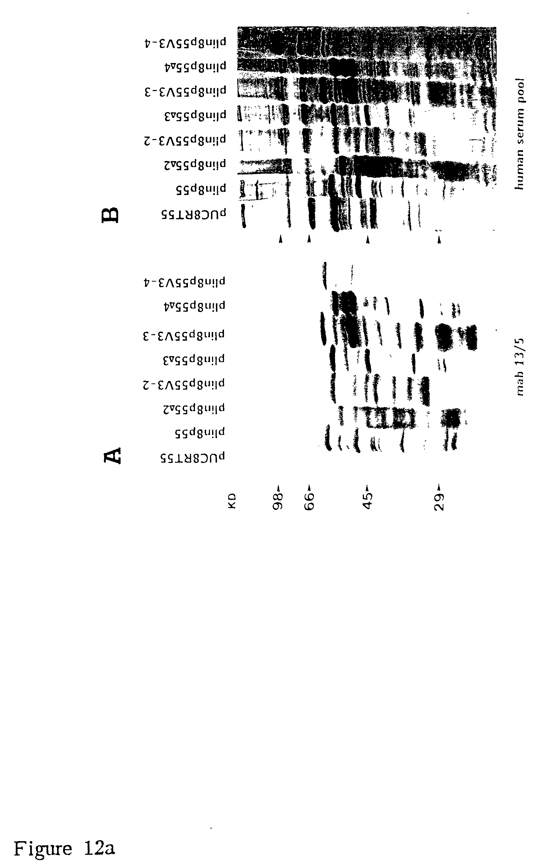

- Fig. 12a:: Expression of three different plin8p55 deletion constructs (plin8p55 2, 3, 4) next to the expression of the respective clones with the inserted consensus sequence of the major HIV-1 neutralizing epitope V3 (plin8p55V3-2,-3,-4). Recombinant bacteria were grown to an optical density of 0,6, induced for 2 h by 2 mM IPTG, lysed in boiling mix and characterized by conventional Western blot analysis. The recombinant p55V3 fusion proteins were specifically detected by monoclonal antibodies directed to p24 (a) and by human serum pool (b).

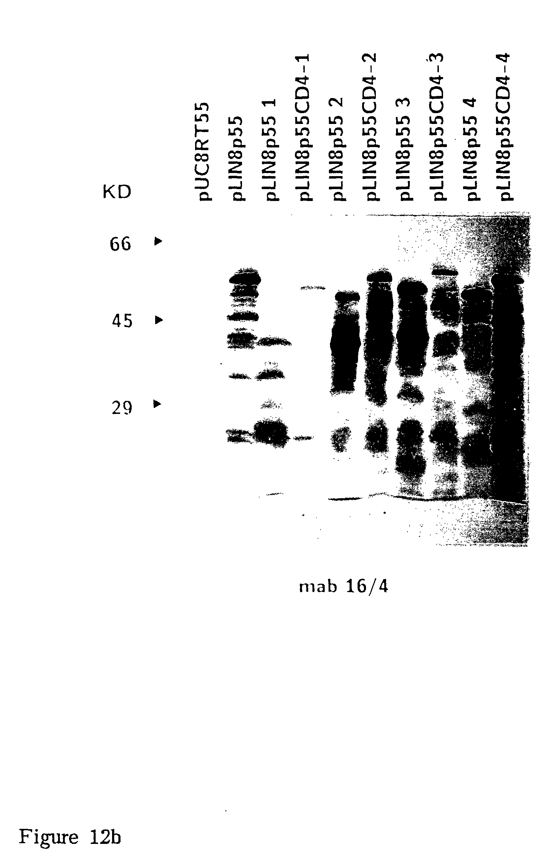

- Fig. 12b:: Expression of three different plin8p55 deletion constructs (plin8p55 1, 2, 3, 4) next to the expression of the respective clones with the inserted CD4-binding region of the HIV-1 gp120 C3 region (plin8p55CD4-1,-2,-3,-4). Recombinant bacteria were grown to an optical density of 0,6, induced for 2 h by 2 mM IPTG, lysed in boiling mix and characterized by conventional Western blot analysis. The recombinant p55CD4 fusion proteins were specifically detected by monoclonal antibodies directed to p24.

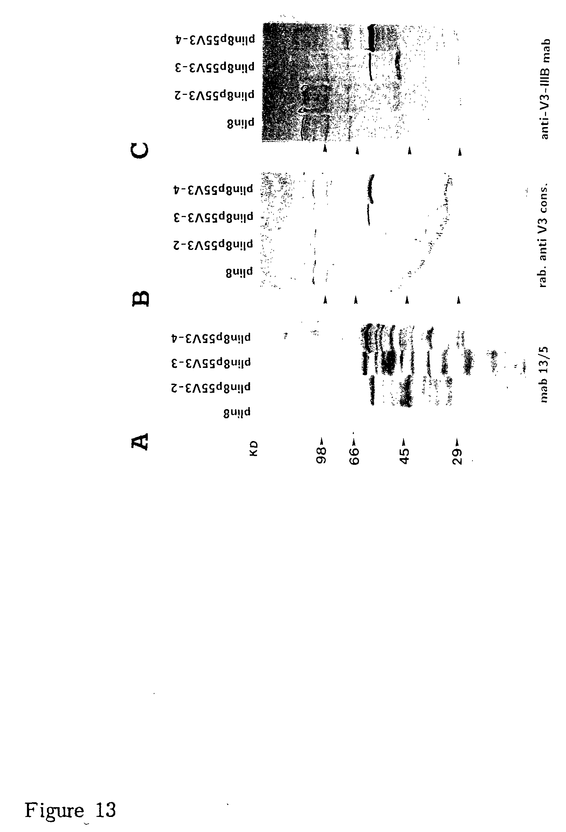

- Fig. 13:: Expression of three different p55/V3 fusion proteins (p55/V3-2,3,4) in E. coli after induction with 2mM IPTG at an O.D. of 0,6. The recombinant p55V3 fusion proteins were specifically detected by monoclonal antibodies directed to p24 (a) and (c) to the V3 epitope of a foreign isolate (IIIb). A polyclonal serum raised against a synthetic peptide, representing the original sequence of the V3 consensus sequence, detected the p55/V3 even better than the Illb-peptide derived monoclonal antibody. The fact, that neither the monoclonal antibody, nor the polyclonal V3 specific serum detect the p55V3-2 protein indicates, that the antigenic context of the V3 region is important for immunological detection.

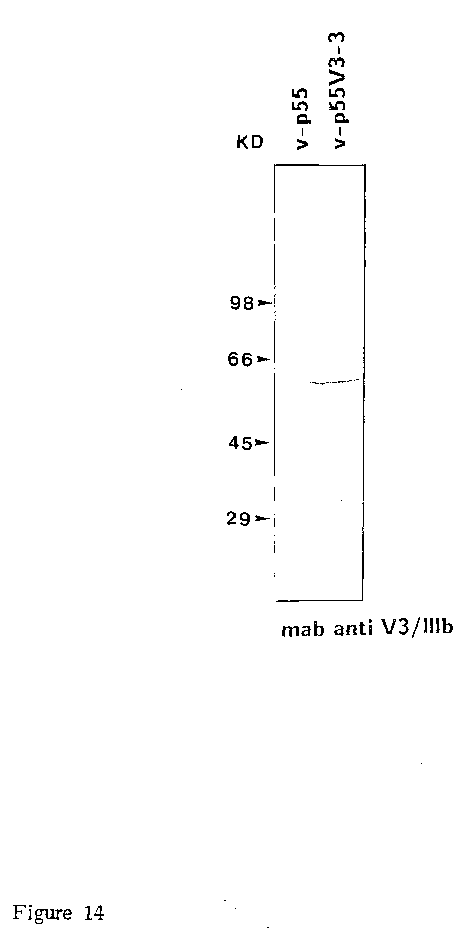

- Fig.14:: CV-1 cells were infected with 10 pfu v-p55V3-3, harvested 2 days after infection and characterized by conventional Western blot analysis using monoclonal antibodies directed to p24 and the IIIb derived V3 region.

The examples illustrate the invention.

### Example 1: Expression of p55 in E. coli (standard methods: Sambrook et al., 1989)

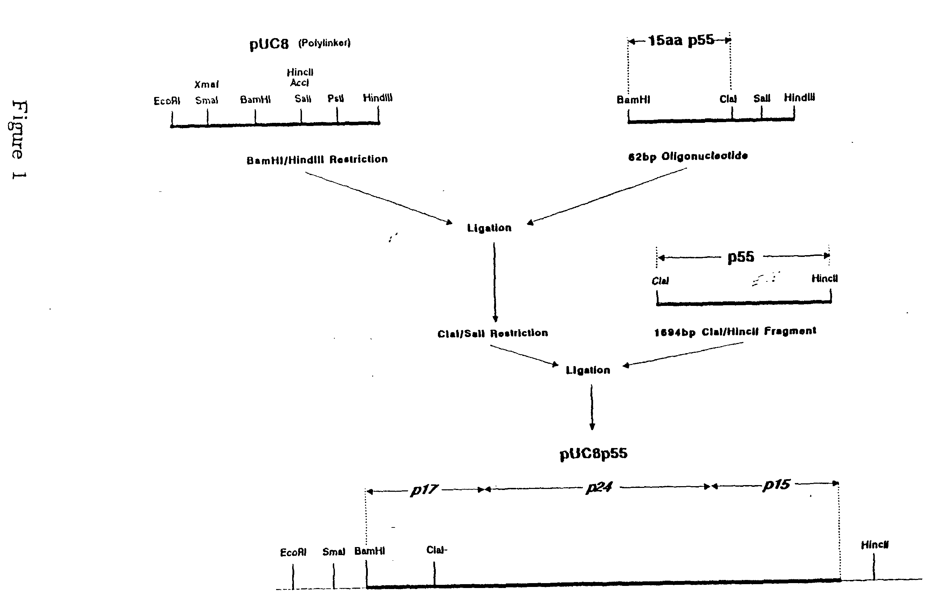

Before p55 could be produced in eukaryotic systems, it had to be cloned and expressed in E. coli. For preliminary expression, the pUC-vector system was chosen. To clone the 5' sequence of the core protein precisely, without longer untranslated sequences, we inserted a synthetic 62 bp oligonucleotide, encoding the N-terminal 15 aa of p55, in frame with the lacZ alpha fragment into the Bam Hl/Hind I II site of the pUC 8 vector. Two 3' in the oligonucleotide localized restriction sites, Clal and Sall, enabled us to complete the gag reading frame by insertion of a 1694 bp p55-ClaI/HincII fragment; fig.1). The expression of the recombinant protein in E. coli after induction with 2mM IPTG resulted in the production of a 55 KD protein proven by conventional Western blot analysis using monoclonal antibodies.

### Example 2: Expression of p55 by recombinant vaccinia viruses

The BamHI/SalI fragment encoding the total p55 reading frame, could be directly subcloned into the vaccinia pAvB transfer-vector and recombined into vaccinia viruses. Expression products were analyzed by conventional Western blot analysis using anti-p24 antibodies (fig.2).

### Example 3: Expression of p55 by recombinant baculo viruses

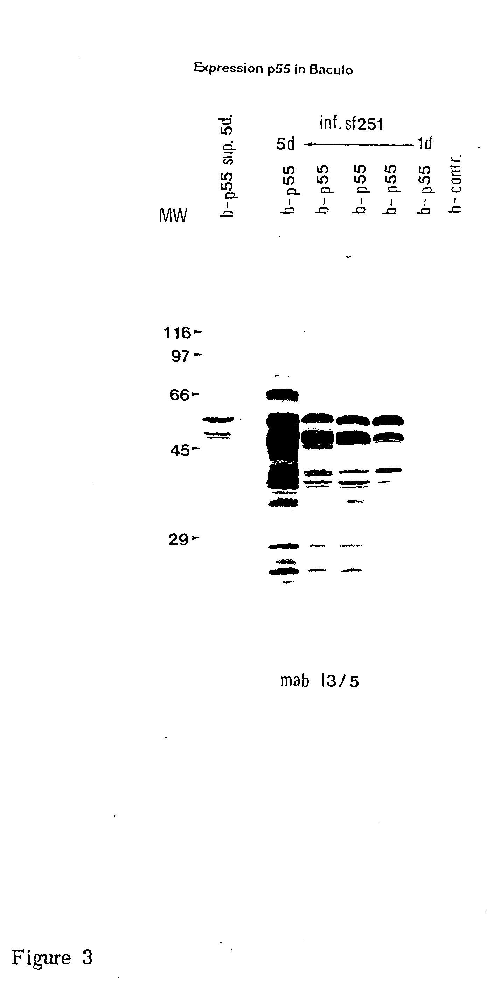

In order to express the core protein by recombinant baculo viruses, it had to be subcloned twice: The BamHI/SalI fragment had to be inserted into the Bam HI/SalI site of pUC19. The p55 coding SacI/SalI fragment was then further subcloned into the SacI/SalI site of a commercial pIC19R vector. After this procedure, the p55 coding sequence could be recloned into a unique BamHI site of the baculo-transfervector pVL941 (Summers et al., 1988). After a recombination event, the core protein was expressed by p55-recombinant baculo viruses in spodoptera frugiperda insect cells. The expression was analyzed by Western blot analysis (fig.3).

### Example 4: Identification of premature p55 particles

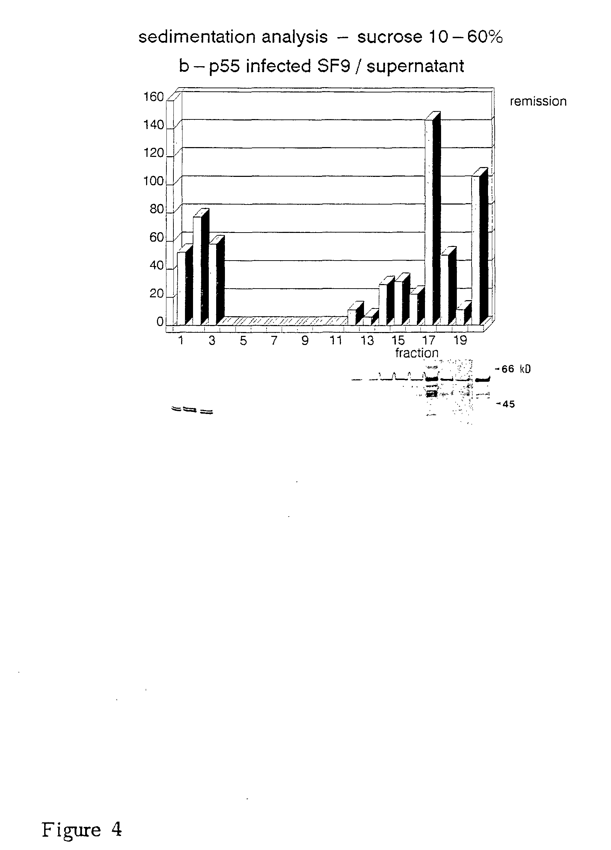

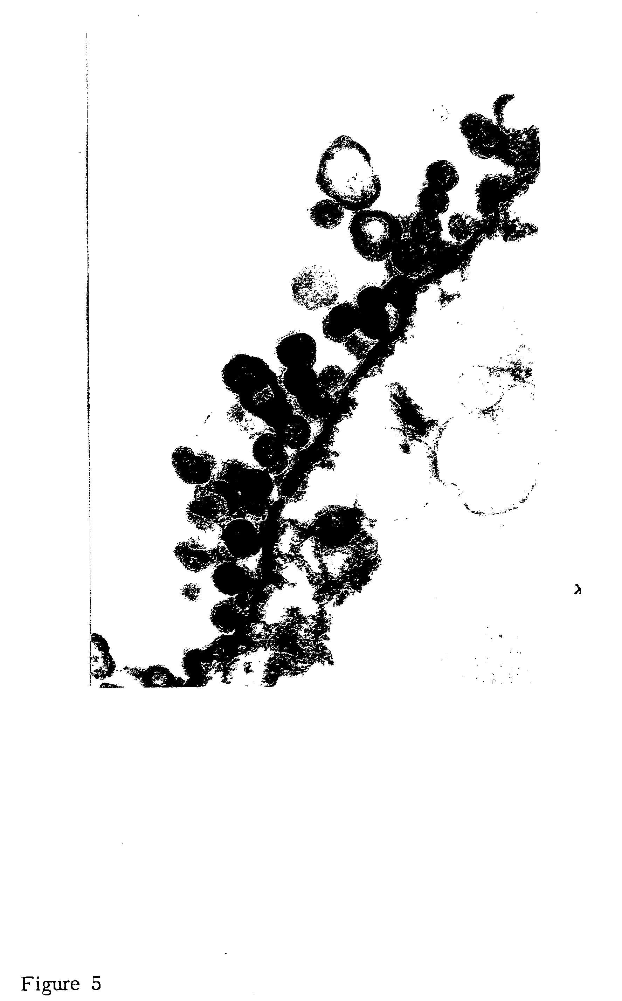

p55-particle formation was characterized by sucrose sedimentation analysis and electron microscopy. Sucrose gradients were centrifuged at 28000 rpm for 150 minutes in an TST 41.14 rotor. 19 fractions were analysed by negative staining and Western blot analysis for p55 particles. Particles accumulated in fraction 17 at a sucrose density of 1.41 (fig.4). Analysis of spodoptora frugiperda cells by ultrathin section electron microscopy identified premature p55 particles budding from the cytoplasmic membran into the culture medium (fig.5).

### Example 5: Computer assisted analysis of p55 secondary structure

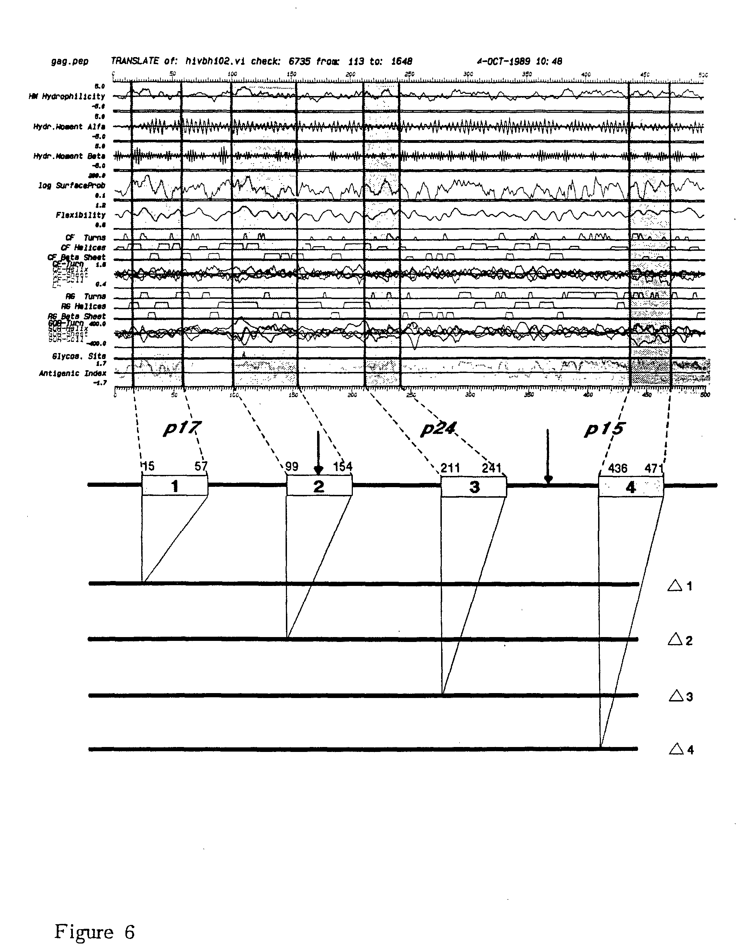

To be able to insert selected epitopes derived from other reading frames than gag into the core protein in a rational manner, several p55 deletion constructs missing a stretch of 120 to 180 bp had to be established on DNA level. We decided, based on computer assisted analysis of the p55 secondary structure, to delete four 30 to 45 amino acids encoding sequences according to criteria of exposition of the respective regions on the surface of the p55 protein (fig. 6). All deletion mutants were derived from the pUC8p55 expession clone.

### Example 6: Construction of the p55 deletion constructs

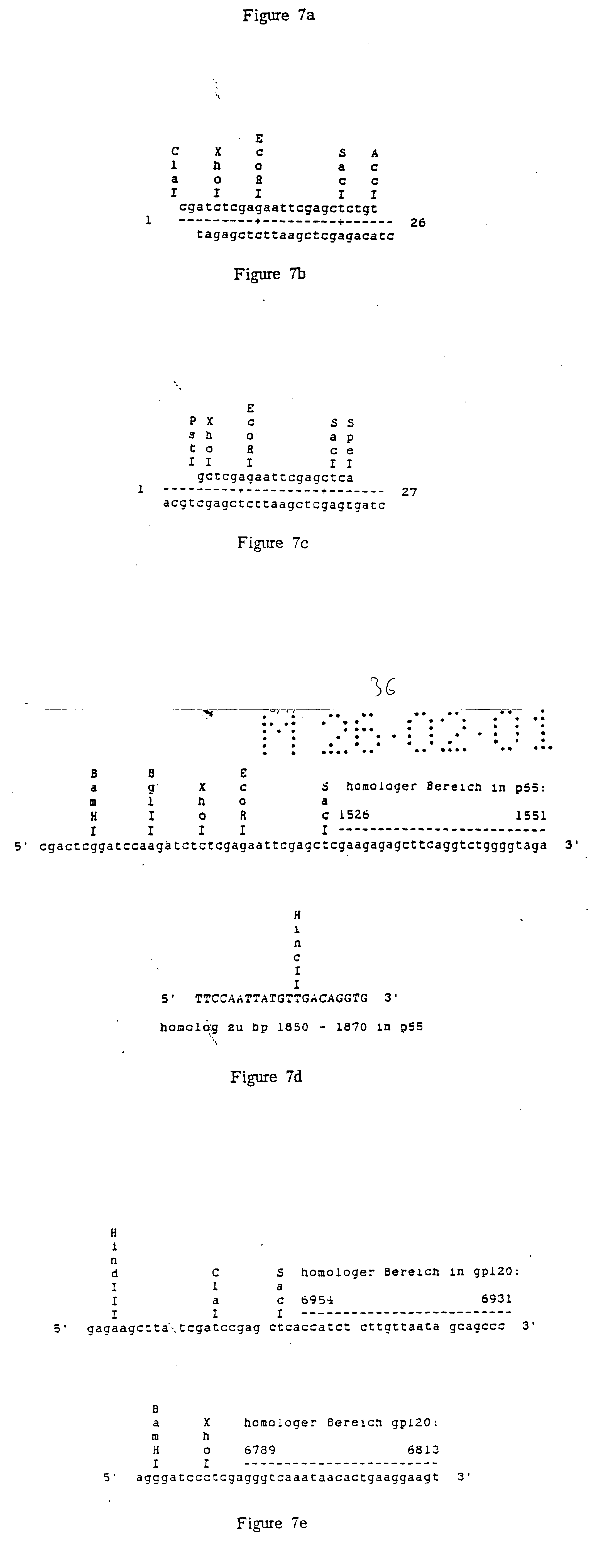

Basis of all four p55 deletion constructs was an especially designed polylinker supplementing the pUC8 derived polylinker. The new linker fragment contained all restriction sites for construction of four different p55 deletion clones, for direct expression in E. coli and for direct subcloning of the final constructs into different transfer vectors, designated for eukaryotic gene expression. The resulting pUC8 derivat was called plin8 (fig.7a).

### Reference 1: p55 deletion construct-1 (plin8p55 1)

The development of the p55 deletion construct-1 based on the pUC19p55 clone described above. To delete two Accl sites, the p55 coding sequence was shortened for the 1100 bp 3'PstI fragment (pUC19p55BP). A 26bp linker fragment, containing two additional restriction sites (SacI/XhoI; fig. 7b) was inserted into the ClaI/AccI sites of the N-terminal p55 coding sequence to achieve compatibility of the described construct to all following p55 deletion clones (pUC19p55BP 1). By this, a 129bp DNA fragment, encoding 43aa was deleted. Afterwards, the p55 coding sequence was completed by the 3'PstI/SalI fragment (pUC19p55 1). The resulting p55 deletion fragment-1 was subcloned into the plin8 vector (plin8p55 1) (fig.8).

### Example 7: p55 deletion construct-2 (plin8p55 2)

The pUC8 derived 300bp BamHI/HindIII fragment, encoding an N-terminal part of p55 was cloned into the BamHI/HindIII site of plin8 (plin8p55BH). Aftenvards, the 1283bp 3'Nsil/Sall fragment was inserted into the PstI/SalI site of plin8p55BH (plin8p55 2) (fig.9).

### Example 8: Development of the deletion construct-3 (plin8p55 3)

A pUC8p55 derived BamHI/SalI fragment, encoding the complete HIV-1 core protein was subcloned into the BamHI/SalI sites of the described plin8 vector (plin8p55). To make the construct compatible to the other p55 deletion clones, a 27bp XhoI/SacI linker fragment was inserted into the Pstl/Spel site of plin8p55. The resulting p55 deletion construct was renamed plin8p55 3 (fig.10).

### Example 9: Development of the deletion construct-4 (plin8p55 4)

The complete pUC8p55 derived gag coding sequence was cloned into the BamHI/SalI site of plin8 (plin8p55). The 3'part (encoding aa 471-512) of the p55 coding sequence was amplified by polymerase chain reaction using a 61 bp 5'primer containing five different restriction sites (BamHI, BglII, Xhol, EcoRI, Sacl) and a 21 bp 3'primer containing a HincII restriction site (fig. 7d). The amplified BglII/SalI fragment was inserted into the BglII/SalI site of the plin8p55 construct (fig.11). In the final plin8p55 4 clone, a 35aa encoding 105bp sequence was deleted.

### Example 10: Compatibility of the four p55 deletion constructs

The developed p55 deletion constructs are compatible with regard to:
1. the insertion of foreign sequences. A defined sequence can be inserted into each of the four casettes without changes in the reading frame.
2. the possibility to directly subclone the completed constructs into vectors appropriate for eukaryotic gene expression.

### Example 11: Expression of the p55 deletion constructs in E. coli

All deletion constructs were expressed in E. coli JM109 after induction with 2mM IPTG. The expression products were verified by conventional Western blot analysis using anti p24 monoclonal antibodies.

### Example 12: Insertion of selected epitopes of other reading frames

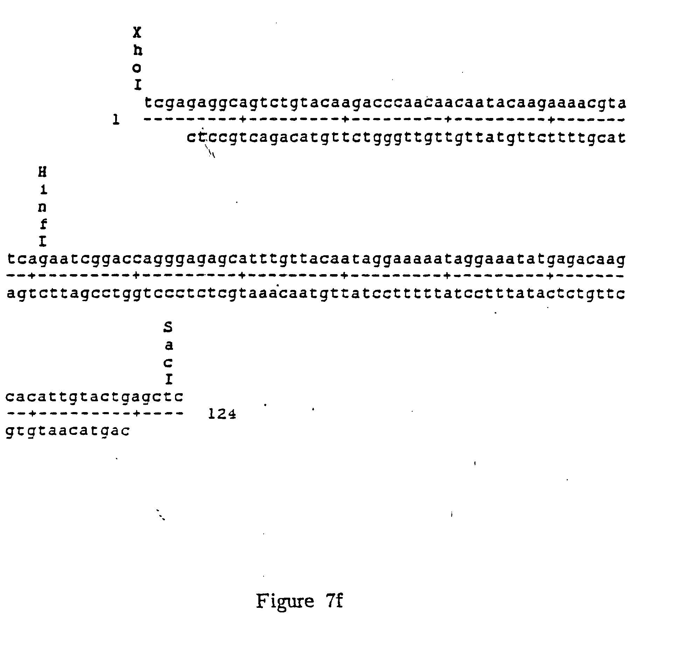

1. The CD4 binding region of the HIV-1 gp120 and the fusogenic region of the gp41 were amplified by PCR reaction. 5'primers contained a Xhol-, 3'primers a Saci site to be able to insert amplified Xhol/ Sacl fragments into the p55 deletion constructs (fig. 7e).
2. A designed consensus sequence of the gp120 major neutralizing epitope V3 was chemically synthesized and integrated into the Xhol/Sacl site of the p55 deletion constructs (fig.7f).

### Example 13: Expression of the completed p55/gp120 fusion proteins in E. coli

The p55/gp120 fusion products described in example 15 were expressed in E. coli after induction with 2 mM IPTG and analyzed by conventional Western blot analysis using anti-p24 monoclonal antibodies and a human serum pool (fig.12A).

### Example 14: Verification of correct expression of an inserted epitope

The expression of the inserted gp120 major neutralizing epitope V3 was verified using a commercial monoclonal antibody directed to the V3 region of the HT-LVIIIb strain by conventional Western blot analysis. A polyclonal monospecific serum raised to the synthetic V3 consensus peptide also recognized the integrated epitope in Western blot analysis.

### Example 15: Subcloning of the completed p55/gp120 constructs in eukaryotic expression vectors

The p55/gp120 constructs were subcloned (i) into the BglII/SalI site of the vaccinia transfer vector pAvB (ii), into the unique BamHI site of the baculo transfer vector pVL941 and (iii) the Xbal/Sall site of the pMD vector for expression of the chimeric proteins in CHO cells.

### Example 16: Expression of the completed p55/gp120 constructs in eukaryotic expression systems

After transfection and recombination of the hybrid genes into the viral DNA (Vaccinia, Baculo), the fusion proteins were expressed by recombinant vaccinia viruses in CV1- and by recombinant baculo viruses in Spodoptera frugiperda cells. The expression products were verified by conventional Western blot and immunofluorescence analysis with specific anti-p24 monoclonal antibodies (fig.14).

### Example 17: Analysis of particle formation

Particle formation was studied by electron microscope using ultrathin sections of glutaraldehyd fixed cells infected with p55/gp120 recombinant vaccinia- or baculo viruses.

### REFERENCES [0053]

Berzofsky, J. A., Bensussan, A., Cease, K. B., Bourge, J. F., Salaun, J. J., Gallo, R. C., Shearer, G. M. and Zagury, 1988, Antigenic peptides recogniced by T lymphozytes from AIDS viral envelope immune humans, Nature, Vol. 334, pp 706
Chan, T. L., Dreesman, G. R., Kanda, P., 1986, Induction of anti HIV neutralizing antibodies by synthetic peptides, Eur. Mol. Biol. Org., Vol. 5, pp 3065
Cianciolo, G. J., Matthews, T. J., Bolognesi, D. P., Snyderman, R., 1980, Macrophage accumulation in mice is inhibited by low molecular weight products from murine leukemia viruses, J. Immunol., Vol. 124, pp 2900-2905
Clayton, L. K., Sieh, M., Pious, D.A., Reinherz, E. L., 1989, Identification of human CD4 residues affecting class II MHC versus HIV-I gp120 binding, Nature, Vol. 339, pp 548-551
Evans D.J., McKeatingJ., Meredith J.M., Burke KL., Katrak K.J., John, A., Ferguson, M., Minor, P.D., Weiss, R.A., Almond, I.W.: An engineered poliovirus chimaera elicts broadly reactive HIV-1 neutralizing antibodies. Nature, 339 (6223), 385-388, 340, Juni 1989.
Golding, H., Robey, F. A., Gates, F. T. et al., 1988, Identification of homologousregions in HIV-1 gp41 and human MHC classll domain, J. Exp. Med., Vol. 167, pp914
Golding, H., Shearer, G., M., Hillman, K. et al., 1989, Common epitope in HIV-1 gp41 and HLA class II elicts immunosuppressive autoantibodies capable of contributing to immune dysfunktion in HIV infected individuals. J. Clin. Invest., Vol. 83, pp1430
Goudsmit, I., Debouck, C., Moloen, R.H., Smit, L., Bakker, M., Asher, D.M., Wolff, A.V., Gibbs, C.J., Gajdusch, D.C. (1988) Human immunodeficiency virus type 1 neutralization epitope with conserved architecture elicts early type specific antibodies in experimentally infected chimpanzees. PNAS-USA, Vol. 85, p. 4478.
Hattori, T., Koito, A., Takatsuki, K., Kido, H., Katunuma, N. (1989) Involvement of tryptase-related cellular proteases in human immunodeficiency virus type 1 infection. FEBS-Lett., Vol. 248, p. 48-52.
Homsy J., Meyer M., Tateno M., Clarkson S., Levy J. A., 1989. The Fc and not CD4 Receptor mediates Antibody Enhancement of HIV Infection in human Cells. Science 244, 1357-1360.
Hu, S.L., Fultz, P.N., Mcclure, H.M., Eichberg, J.W., Thomas, E.K., Zarling, J., Snighal, M.C., Kosowski, S.G., Swenson, R.B., Anderson, D.C., Todaro, G. (1987) Effect of Immunization with a vaccinia-HIV env recombinant on HIV infection of chimpanzees. Nature, Vol. 328, p. 721.
Kowalski, M., Potz, J., Basiripour, L., Dorfmann, T., Goh, W. C., Terwilliger, E., Dayton, A., Rosen, C., Haseltine, W., Sodroski, J., 1987. Functional Regions of the Envelope Glycoprotein of Human Immunodeficiency Virus Type 1. Science 237, 1351-1355.
Krohn, K. J. E., Lusso, E., Gallo, R. C. et al., 1988, Identification of two T cell specific epitopes in the conserved regions of human immunodeficiency virus glycoprotein. In: vaccines 88-new chemical and genetic approaches to vaccination: Prevention of AIDS and other viral, bacterial and parasitic disease eds. Ginsberg, H., Brown, F., Renner, T. A., Chanock, R. M., Could spring harbour, New York: Cold spring harbour laboratory, pp. 357
Lancavecchia, A., Roosney, E., Gregory, T., Berman, P., Agrignani, S., 1988, T cells can present antigens such as HIV gp120 targeted to their own surface molecules, Nature, Vol. 334, pp530
Lasky, L. A., Nakamura, G., Smith, D. H., Fennie, C., Shimasaki, C., Patzer, E., Bermann, P., Gregory, T., Capon, D. J., (1987). Delineation of a Region of the Human Immunodeficiency Virus Type 1 gp120 Glycoprotein Critical for Interaktion with the CD4 Receptor. Cell 50, 975-985.
Lyerly H. K., Matthews T. J., Langlois, A. J., Bolognesi, D. P., Weinhold, K. J., 1987, Human T-cell lymphotropic virus IIIB glycoprotein bound to CD4 determinants on normal lymphocytes and expressed by infected cells serves as a target for immune attack, PNAS-USA, Vol. 84, pp 4601
Lyerly H. K., Reed D. L., Matthews T. J., 1988, Anti gp120 antibodies from HIV seropositive individuals mediate broad reactive anti-HIV ADCC, AIDS Res Hum Retrovirus, Vol. 3, pp 409
McClure, M. O., Sattenau, Q. J., Beverly, P. C. L., Hearn, J. P., Fitzgerald, A. K., Zuckermann, A. J., Weiss, R. A., (1987). HIV Infection of Primate Lymphocytes and Conservation of the CD4 Receptor. Nature (London) 330, 487-489.
Milich, D.R., Huges, J.L., McLachlan, A., Thornton, G.B., Moriarty, A. (1988) Hepatitis B synthetic immunogen comprisid of nucleocapsid T-cell sites and an envelope B-cell epitope. PNAS-USA, Vol. 85, p. 1610.
Modrow, S., Hahn, B. H., Shaw, G. M., Gallo, R. C., Wong-Stahl, F., Wolf, H., 1987. Computer-assisted Analysis of Envelope Protein Sequences of seven Human Immunodeficiency Virus Isolates: Prediction of Antigenic Epitopes in Conserved and Variable Regions. J Virol 61, 570-578.
Nara, P.L., Robey, W.G., Pyle, S.W., Hatch, W.C., Dunlop, N.M., Bess, I.W. Kelliher, J.C., Arthur, L.O., Fischinger, P.J. (1988) Purified envelope glycoproteins from human immunodeficiency virus type 1 variants induce individual, type specific neutralizing antibodies. J. Virol., Vol. 62, p. 2622.
Nixon, D. F., Townsend, A. R. M., Elvin, J. G., Rizza, C., R., Gallwey, J. and Mc Micehael, A. J., 1988, HIV-1 gag specific cytotoxic T- lymphozytes defined with recombinant vaccinia virus and synthetic peptides, Nature, Vol. 336, pp. 484
Palker, T.J., Clark, M.E., Langlois, A.J., Matthews, T.J., Weinhold, K.J., Randall, R.R., Bolognesi, D.P., Haynes, B.F. (1988) Type specific neutralization of the human immunodeficiency virus with antibodies to env-encoded synthetic peptides. PNAS-USA, Vol. 85, p. 1932.
Papsidero, L.D., Sheu, M., Ruscetti, F.W. (1989) Human immunodeficiency virus type 1-neutralizing monoclonal antibodies which react with p17 core protein:. characterization and epitope mapping. J. virol., Vol. 63, p.267.
Reiher, W. E., Blalock, J. E., Brunck, T. K.; 1989, Sequence homology between aquired immunodeficiency syndrome virus envelope protein and interleukin 2, PNAS-USA, Vol. 83, pp9188
Robinson, W.E., Mantefiori, D.C., Mitchell, W.M., Bruice, A.M., Altor, H.J., Dreesman, G.R., Eichberg, J.W., 1989, Antibody dependent enhancement on human immunodeficiency virus type 1 infection in vitro by serum from HIV-1 infected and passively immunized chimpanzees, Proc. NaTl. Acad. Sci. USA, Vol. 86, pp 4710
Schrier, R. D., Gnann, J. W., Landes, R., Lockshin, C., Richman, D., McCutchan, A., Kennedy, C., Oldstone, M. B. and Nelson, J. A., 1989, T-cell recognition of HIV synthetic peptides in a natural infection, J. Immunol. pp. 1166
Siliciano, R. F., Lawton, T., Knall, C. et al., 1988, Analysis of host virus interactions in AIDS with antigp120 T cell clones: effect on HIV sequence variation and a mechanism for CD4+ cell depletion, Cell, Vol. 54, pp 561
Siliciano, R. F., Lawton, T., Knall, C., Karr, R. W., Berman, B., Gregory, T. and Reinherz, E. L., 1988, Analysis of host-virus interactions In AIDS with anti gp120 T-cell clones: effect of HIV sequence variation and a mechanism for CD4+ cell depletion, Cell, Vol. 54, pp. 561
Skinner M. A., Langlois, A. J., McDanal, C. B., Mc Dougal, J. S., Bolognesi, D. P., Mathews, T.,J., 1988, Neutralizing antibodies to immunodominant enve!ope sequenz do not prevent gp120 binding to CD4, J. Virol., Vol. 62, pp4195
Sun, N-C., Ho, D. D., Sun, C. R. Y., Liou, R-S., Gordon, W., Fung, M. S. C., Li, X-L., Ting, R. C., Lee, T-H., Chang, N. T., Chang, T-W., (1989). Generation and Characterisation of Monoclonal Antibodies to the Putative CD4-Binding Domain of Human Immunodeficiency Virus Type 1 gp120. J Virol 63, 3579-3585.
Takahashi, H., Cohen, J., Hosmalin, A., Cease K. B., Houghten, R., Cornette, I.L., De Lisi, C., Moss, B., Germain, R.N., Berzofsky, I.A. (1988) An immunodominant epitope of the human immunodeficiency virus envelope glycoprotein gp120 recognized by class I major histocompatibility complex molecule-restricted murine cytotoxic T-lymphocytes. PNAS-USA, Vol. 85, p. 3105.
Takeda, A., Tuazon, C. U., Ennis, F. A., 1988, Antibody enhanced infection by HIV-1 via FC-receptor mediated entry. Science, Vol. 242, pp 580
Vornhagen, R., Motz, M., Soutschec-Bauer E., Pichler W., Siegl G., Hinderer W., Wolf H., Sonneborn H.-H., 1989, The determination of additional serological markers concerning HIV-infection using recombinant viral antigens of all HIV-1 reading frames, V. int. conf. on AIDS, (abs. W.B.P.118) abs. M.C.O.7)
Walker, D. B., Birch-Limberger, K., Fischer, L., Young, R., Moss, B. and Schooley, R. T. 1989, Longterm culture and fine antigen specificity of HIV-1 reverse transcriptase-specific cytotoxic T-lymphocytes (CTL) clones. V int. Conf. on AIDS, Montreal, (abs. W.C.O.43
Walker, D.B., Flexner, C.W., Paradis, T.J., Fuller, T. C., Hirsch, M.S., Schooley, R.T., Moss, B., 1988, HIV-1 Reverse Transcriptase is a target for cytotoxic T-lymphocytes in infected individuals.
Weinhold, K. J., Lyerly, H. K., Stanley, S. D., Austin, A. A., Matthews, T. J., Bolognesi, D. P., 1989, HIV-1 gp120-mediated immune suppression and lymphocyte destruction in the absence of viral infection, J. Immunol., Vol. 142, pp 3091-3097
Wolf, H., Modrow, S., Motz, M., Jameson, B., Herrmann, G. and Förtsch B., 1988, An integrated family of aminoacid sequence analysis programs, Cabios, Vol. 4, pp 187
Wolf, H., Modrow, S., Soutschek, E., Motz, M., Grunow, R., Döbl, H., v. Baehr, R. (1990) Herstellung, Kartierung und biologische Charakterisierung von monoklonalen Antikörpern gegen das Core protein (p24) des humanen Immundefizienz-Virus. AIFO, Vol. 1.

## Claims (Claims for the following Contracting State(s): AT, BE, CH, DE, DK FR, GB, IT, LI, LU, NL, TS, SE)

1. A modified retroviral gag polypeptide which forms a particle wherein said gag polypeptide contains at least one amino acid sequence representing a non-gag polypeptide antigenic determinant, wherein the gag polypeptide is p55 of HIV-1, wherein said amino acid sequence substitutes (a) region(s) of said gag polypeptide, said region(s) being capable of presenting the inserted amino acid sequence to the immune system so as to elicit an immune response, and wherein said region is located at amino acid positions 99 to 154, 211 to 241, or 436 to 471 or at any combination thereof.

2. The modified retroviral gag polypeptide according to claim 1, wherein the non-gag polypeptide antigenic determinant is
(a) at least a part of the CD4-binding domain of gp120;
(b) at least a part of the variable region 3 of gp120; or
(c) at least a part of the fusogenic region of gp41.

3. A DNA sequence encoding the modified retroviral gag polypeptide according to claim 1 or 2.

4. The DNA sequence according to claim 3, wherein the DNA sequence encoding the amino acid sequence is inserted via a linker.

5. A recombinant vector containing a DNA sequence according to claim 3 or 4.

6. A recombinant virus containing a DNA sequence according to claim 3 or 4.

7. The recombinant virus according to claim 6, which is a vaccinia virus.

8. A host organism which is transformed by a recombinant vector according to claim 5, or a recombinant virus according to claim 6 or 7.

9. A method for the production of a modified retroviral gag polypeptide according to claim 1 or 2, which comprises cultivating a host organism according to claim 8 under suitable conditions and recovering the expression product from the medium.

10. The method according to claim 9, wherein said host organism is an insect cell, preferably a Spodoptera frugiperda cell and wherein said virus with which said insect cell is transformed is a recombinant insect virus, preferably a baculo virus.

11. The method according to claim 9, wherein said host cell is an insect cell, preferably a Drosophila Schneider cell, and wherein said recombinant vector with which said insect cell is transformed is stably replicable in insect cells.

12. A vaccine containing a recombinant virus according to claim 6 or 7 or a modified retroviral gag polypeptide according to claim 1 or 2, optionally in combination with a pharmaceutically acceptable carrier and/or diluent.

## Claims (Claims for the following Contracting State(s): GR)

1. A modified retroviral gag polypeptide which forms a particle, wherein said gag polypeptide contains at least one amino acid sequence representing a non-gag polypeptide antigenic determinant, wherein the gag polypeptide is p55 of HIV-1, wherein said amino acid sequence substitutes (a) region(s) of said gag polypeptide, said region(s) being capable of presenting the inserted amino acid sequence to the immune system so as to elicit an immune response, and wherein said region is located at amino acid positions 99 to 154, 211 to 241, or 436 to 471 or at any combination thereof.

2. The modified retroviral gag polypeptide according to claim 1, wherein the non-gag polypeptide antigenic determinant is
(a) at least a part of the CD4-binding domain of gp120;
(b) at least a part of the variable region 3 of gp120; or
(c) at least a part of the fusogenic region of gp41.

3. A DNA sequence encoding the modified retroviral gag polypeptide according to claim 1 or 2.

4. The DNA sequence according to claim 3, wherein the DNA sequence encoding the amino acid sequence is inserted via a linker.

5. A recombinant vector containing a DNA sequence according to claim 3 or 4.

6. A recombinant virus containing a DNA sequence according to claim 3 or 4.

7. The recombinant virus according to claim 6, which is a vaccinia virus.

8. A host organism which is transformed by a recombinant vector according to claim 5, or a recombinant virus according to claim 6 or 7.

9. A method for the production of a modified retroviral gag polypeptide according to claim 1 or 2, which comprises cultivating a host organism according to claim 8 under suitable conditions and recovering the expression product from the medium.

10. The method according to claim 9, wherein said host organism is an insect cell, preferably a Spodoptera frugiperda cell and wherein said virus with which said insect cell is transformed is a recombinant insect virus, preferably a baculo virus.

11. The method according to claim 9, wherein said host cell is an insect cell, preferably a Drosophila Schneider cell, and wherein said recombinant vector with which said insect cell is transformed is stably replicable in insect cells.

12. Use of a recombinant virus according to claim 6 or 7 or a modified retroviral gag polypeptide according to claim 1 or 2, optionally in combination with a pharmaceutically acceptable carrier and/or diluent for the preparation of a vaccine.

## Claims (Claims for the following Contracting State(s): ES)

1. A method for the production of a DNA sequence encoding a modified retroviral gag polypeptide which forms a particle, wherein said gag polypeptide contains at least one amino acid sequence representing a non-gag polypeptide antigenic determinant wherein said gag polypeptide encoding DNA sequence is derived from the retrovirus HIV-1, wherein said DNA sequence encoding said amino acid sequence substitutes (a) region(s) of said DNA sequence encoding said gag polypeptide, said region(s) being capable of presenting the amino acid sequence encoded by the inserted DNA sequence to the immune system so as to elicit an immune response, and wherein said region is located at amino acid positions 99 to 154, 211 to 241, or 436 to 471 or at any combination thereof, said method comprising the substitution of at least one codon of a DNA sequence encoding said gag polypeptide by a DNA sequence encoding a non-gag amino acid sequence.

2. The method according to claim 1, wherein the DNA sequence encoding the additional amino acid sequence is inserted via a linker.

3. The method according to claim 1 or 2, wherein the non-gag polypeptide antigenic determinant is
(a) at least a part of the CD4-binding domain of gp120;
(b) at least a part of the variable region 3 of gp120; or
(c) at least a part of the fusogenic region of gp41.

4. A method for the production of a recombinant vector comprising the insertion of a DNA sequence according to any one of claims 1 to 3 into a vector molecule.

5. A method for the production of a recombinant virus comprising the steps of inserting a DNA sequence according to any one of claims 1 to 3 into a viral DNA molecule.

6. The method according to claim 5, wherein the recombinant virus is a vaccinia virus.

7. A method for the production of a host carrying a recombinant vector obtainable by a method according to claim 4 or a recombinant virus obtainable by a method according to claim 5 or 6, said method comprising the incorporation of said recombinant vector or said recombinant virus into a suitable host.

8. A method for the production of a modified retroviral gag polypeptide which is encoded by a DNA sequence according to any one of claims 1 to 3, comprising the steps of cultivating a host obtainable by a method according to claim 7 under suitable conditions and recovering said polypeptide from the medium.

9. A method according to claim 8, wherein the polypeptides form particles.

10. The method according to claim 8 or 9, wherein said host organism is an insect cell, preferably a Spodoptera frugiperda cell and wherein said virus with which said insect cell is transformed is a recombinant insect virus, preferably a baculo virus.

11. The method according to claim 8 or 9, wherein said host cell is an insect cell, preferably a Drosophila Schneider cell, and wherein said recombinant vector with which said insect cell is transformed is stably replicable in insect cells.

## Patentansprüche (Patentansprüche für folgende(n) Vertragsstaat(en): AT, BE, CH, DE, DK FR, GB, IT, LI, LU, NL, TS, SE)

1. Modifiziertes retrovirales gag-Polypeptid, das ein Partikel bildet, wobei das gag-Polypeptid mindestens eine Aminosäuresequenz enthält, die eine nicht-gag-Polypeptid-antigene Determinante darstellt, wobei das gag-Polypeptid p55 von HIV-1 ist, wobei die Aminosäuresequenz (einen) Bereich(e) des gag-Polypeptids ersetzt, der (die) die eingefügte Aminosäuresequenz dem Immunsystem präsentieren kann/können, so daß eine Immunantwort hervorgerufen wird, und wobei der Bereich in den Aminosäurepositionen 99 bis 154, 211 bis 241 oder 436 bis 471 oder jeder Kombination davon liegt.

2. Modifiziertes retrovirales gag-Polypeptid nach Anspruch 1, wobei die nicht-gag-Polypeptid-antigene Determinante
(a) mindestens ein Teil der CD4-bindenden Domäne von gp120;
(b) mindestens ein Teil des variablen Bereichs 3 von gp120; oder
(c) mindestens ein Teil des fusogenen Bereichs von gp41 ist.

3. DNA-Sequenz, die das modifizierte retrovirale gag-Polypeptid nach Anspruch 1 oder 2 codiert.

4. DNA-Sequenz nach Anspruch 3, wobei die DNA-Sequenz, die die Aminosäuresequenz codiert, über einen Linker eingefügt ist.

5. Rekombinanter Vektor, der eine DNA-Sequenz nach Anspruch 3 oder 4 beinhaltet.

6. Rekombinantes Virus, das eine DNA-Sequenz nach Anspruch 3 oder 4 beinhaltet.

7. Rekombinantes Virus nach Anspruch 6, das ein Vaccinia-Virus ist.

8. Wirtsorganismus, der mit einem rekombinanten Vektor nach Anspruch 5 oder einem rekombinanten Virus nach Anspruch 6 oder 7 transformiert ist.

9. Verfahren zur Herstellung eines modifizierten retroviralen gag-Polypeptids nach Anspruch 1 oder 2, das das Züchten eines Wirtsorganismus nach Anspruch 8 unter geeigneten Bedingungen und die Gewinnung des Expressionsprodukts aus dem Medium umfaßt.

10. Verfahren nach Anspruch 9, wobei der Wirtsorganismus eine Insektenzelle ist, vorzugsweise eine Spodoptera frugiperda-Zelle, und wobei das Virus, mit dem die Insektenzelle transformiert ist, ein rekombinantes Insektenvirus ist, vorzugsweise ein Baculovirus.

11. Verfahren nach Anspruch 9, wobei die Wirtszelle eine Insektenzelle ist, vorzugsweise eine Drosophila Schneider-Zelle, und wobei der rekombinante Vektor, mit dem die Insektenzelle transformiert ist, in Insektenzellen stabil replizierbar ist.

12. Impfstoff, der ein rekombinantes Virus nach Anspruch 6 oder 7 oder ein modifiziertes retrovirales gag-Polypeptid nach Anspruch 1 oder 2 beinhaltet, gegebenenfalls in Kombination mit einem pharmazeutisch verträglichen Träger und/oder Verdünnungsmittel.

## Patentansprüche (Patentansprüche für folgende(n) Vertragsstaat(en): GR)

1. Modifiziertes retrovirales gag-Polypeptid, das ein Partikel bildet, wobei das gag-Polypeptid mindestens eine Aminosäuresequenz enthält, die eine nicht-gag-Polypeptid-antigene Determinante darstellt, wobei das gag-Polypeptid p55 von HIV-1 ist, wobei die Aminosäuresequenz (einen) Bereich(e) des gag-Polypeptids ersetzt, der (die) die eingefügte Aminosäuresequenz dem Immunsystem präsentieren kann/können, so daß eine Immunantwort hervorgerufen wird, und wobei der Bereich in den Aminosäurepositionen 99 bis 154, 211 bis 241 oder 436 bis 471 oder jeder Kombination davon liegt.

2. Modifiziertes retrovirales gag-Polypeptid nach Anspruch 1, wobei die nicht-gag-Polypeptid-antigene Determinante
(a) mindestens ein Teil der CD4-bindenden Domäne von gp120;
(b) mindestens ein Teil des variablen Bereichs 3 von gp120; oder
(c) mindestens ein Teil des fusogenen Bereichs von gp41 ist.

3. DNA-Sequenz, die das modifizierte retrovirale gag-Polypeptid nach Anspruch 1 oder 2 codiert.

4. DNA-Sequenz nach Anspruch 3, wobei die DNA-Sequenz, die die Aminosäuresequenz codiert, über einen Linker eingefügt ist.

5. Rekombinanter Vektor, der eine DNA-Sequenz nach Anspruch 3 oder 4 beinhaltet.

6. Rekombinantes Virus, das eine DNA-Sequenz nach Anspruch 3 oder 4 beinhaltet.

7. Rekombinantes Virus nach Anspruch 6, das ein Vaccinia-Virus ist.

8. Wirtsorganismus, der mit einem rekombinanten Vektor nach Anspruch 5 oder einem rekombinanten Virus nach Anspruch 6 oder 7 transformiert ist.

9. Verfahren zur Herstellung eines modifizierten retroviralen gag-Polypeptids nach Anspruch 1 oder 2, das das Züchten eines Wirtsorganismus nach Anspruch 8 unter geeigneten Bedingungen und die Gewinnung des Expressionsprodukts aus dem Medium umfaßt.

10. Verfahren nach Anspruch 9, wobei der Wirtsorganismus eine Insektenzelle ist, vorzugsweise eine Spodoptera frugiperda-Zelle, und wobei das Virus, mit dem die Insektenzelle transformiert ist, ein rekombinantes Insektenvirus ist, vorzugsweise ein Baculovirus.

11. Verfahren nach Anspruch 9, wobei die Wirtszelle eine Insektenzelle ist, vorzugsweise eine Drosophila Schneider-Zelle, und wobei der rekombinante Vektor, mit dem die Insektenzelle transformiert ist, in Insektenzellen stabil replizierbar ist.

12. Verwendung eines rekombinanten Virus nach Anspruch 6 oder 7 oder eines modifizierten retroviralen gag-Polypeptids nach Anspruch 1 oder 2, gegebenenfalls in Kombination mit einem pharmazeutisch verträglichen Träger und/oder Verdünnungsmittel zur Herstellung eines Impfstoffs.

## Patentansprüche (Patentansprüche für folgende(n) Vertragsstaat(en): ES)

1. Verfahren zur Herstellung einer DNA-Sequenz, die ein modifiziertes retrovirales gag-Polypeptid codiert, das ein Partikel formt, wobei das gag-Polypeptid mindestens eine Aminosäuresequenz enthält, die eine nicht-gag-Polypeptid-antigene Determinante darstellt, wobei die gag-Polypeptidcodierende DNA-Sequenz von dem Retrovirus HIV-1 stammt, wobei die die Aminosäuresequenz codierende DNA-Sequenz (einen) Bereich(e) der DNA-Sequenz ersetzt, der (die) das gag-Polypeptid codiert, wobei der (die) Bereich(e) dem Immunsystem die von der eingefügten DNA-Sequenz codierte Aminosäuresequenz präsentieren kann/können, so daß eine Immunantwort hervorgerufen wird, und wobei der Bereich in den Aminosäurepositionen 99 bis 154, 211 bis 241 oder 436 bis 471 oder jeder Kombination davon liegt, wobei das Verfahren den Austausch von mindestens einem Codon einer das gag-Polypeptid codierenden DNA-Sequenz durch eine eine nicht-gag-Aminosäuresequenz codierende DNA-Sequenz umfaßt.

2. Verfahren nach Anspruch 1, wobei die die zusätzliche Aminosäuresequenz codierende DNA-Sequenz über einen Linker eingefügt ist.

3. Verfahren nach Anspruch 1 oder 2, wobei die nicht-gag-Polypeptid-antigene Determinante
(a) mindestens ein Teil der CD4-bindenden Domäne von gp120;
(b) mindestens ein Teil des variablen Bereichs 3 von gp120; oder
(c) mindestens ein Teil des fusogenen Bereichs von gp41 ist.

4. Verfahren zur Herstellung eines rekombinanten Vektors, das das Einfügen einer DNA-Sequenz nach einem der Ansprüche 1 bis 3 in ein Vektormolekül umfaßt.

5. Verfahren zur Herstellung eines rekombinanten Virus, das die Schritte des Einfügens einer DNA-Sequenz nach einem der Ansprüche 1 bis 3 in ein virales DNA-Molekül umfaßt.

6. Verfahren nach Anspruch 5, wobei das rekombinante Virus ein Vaccinia-Virus ist.

7. Verfahren zur Herstellung eines Wirts, der einen durch ein Verfahren nach Anspruch 4 erhältlichen rekombinanten Vektor oder ein durch ein Verfahren nach Anspruch 5 oder 6 erhältliches rekombinantes Virus trägt, wobei das Verfahren das Einbringen des rekombinanten Vektors oder des rekombinanten Virus in einen geeigneten Wirt umfasst.

8. Verfahren zur Herstellung eines modifizierten retroviralen gag-Polypeptids, das durch eine DNA-Sequenz nach einem der Ansprüche 1 bis 3 codiert wird, umfassend die Schritte der Züchtung eines durch ein Verfahren nach Anspruch 7 erhältlichen Wirts unter geeigneten Bedingungen und die Gewinnung des Polypeptids aus dem Medium.

9. Verfahren nach Anspruch 8, wobei die Polypeptide Partikel bilden.

10. Verfahren nach Anspruch 8 oder 9, wobei der Wirtsorganismus eine Insektenzelle ist, vorzugsweise eine Spodoptera frugiperda-Zelle, und wobei das Virus, mit dem die Insektenzelle transformiert ist, ein rekombinantes Insektenvirus ist, vorzugsweise ein Baculovirus.

11. Verfahren nach Anspruch 8 oder 9, wobei die Wirtszelle eine Insektenzelle ist, bevorzugt eine Drosophila Schneider-Zelle, und wobei der rekombinante Vektor, mit dem die Insektenzelle transformiert ist, in Insektenzellen stabil replizierbar ist.

## Revendications (Revendications pour l'(les) Etat(s) contractant(s) suivant(s): AT, BE, CH, DE, DK FR, GB, IT, LI, LU, NL, TS, SE)

1. Polypeptide gag rétroviral modifié formant une particule, dans lequel ledit polypeptide gag contient au moins une séquence d'aminoacides représentant un déterminant antigénique polypeptidique non gag, dans lequel le polypeptide gag est p55 de VIH-1, dans lequel ladite séquence d'aminoacides remplace une (des) région(s) dudit polypeptide gag, ladite (lesdites) région(s) étant capable(s) de présenter au système immunitaire la séquence d'aminoacides insérée, de manière à déclencher une réponse immunitaire, et dans lequel ladite région est localisée aux positions d'aminoacides 99 à 154, 211 à 241, ou 436 à 471, ou à une.combinaison quelconque de celles-ci.

2. Polypeptide gag rétroviral modifié, selon la revendication 1, dans lequel le déterminant antigénique polypeptidique non-gag est :
(a) au moins une partie du domaine de liaison à CD4 de gp 120 ;
(b) au moins une partie de la région variable 3 de gp 120 ; ou
(c) au moins une partie de la région fusiogène de gp 41.

3. Séquence d'ADN codant pour le polypeptide gag rétroviral modifié, selon la revendication 1 ou 2.

4. Séquence d'ADN selon la revendication 3, dans laquelle la séquence d'ADN codant pour la séquence d'aminoacides est insérée par l'intermédiaire d'un lieur.

5. Vecteur recombinant contenant une séquence d'ADN selon la revendication 3 ou 4.

6. Virus recombinant contenant une séquence d'ADN selon la revendication 3 ou 4.

7. Virus recombinant selon la revendication 6, qui est un virus de la vaccine.

8. Organisme hôte qui est transformé par un vecteur recombinant selon la revendication 5 ou un virus recombinant selon la revendication 6 ou 7.

9. Procédé pour la production d'un polypeptide gag rétroviral modifié, selon la revendication 1 ou 2, comprenant la culture d'un organisme hôte selon la revendication 8, dans des conditions appropriées, et la récupération du produit d'expression à partir du milieu.

10. Procédé selon la revendication 9, dans lequel ledit organisme hôte est une cellule d'insecte, de préférence une cellule de *Spodoptera frugiperda*, et dans lequel ledit virus par lequel ladite cellule d'insecte est transformée est un virus d'insecte recombinant, de préférence un baculovirus.

11. Procédé selon la revendication 9, dans lequel la cellule hôte est une cellule d'insecte, de préférence une cellule Schneïder de Drosophila, et dans lequel ledit vecteur recombinant par lequel ladite cellule d'insecte est transformée est réplicable de façon stable dans des cellules d'insectes.

12. Vaccin contenant un virus recombinant selon la revendication 6 ou 7, ou un polypeptide gag rétroviral modifié, selon la revendication 1 ou 2, éventuellement en association avec un véhicule et/ou diluant pharmaceutiquement acceptable.

## Revendications (Revendications pour l'(les) Etat(s) contractant(s) suivant(s): ES)

1. Procédé pour la production d'une séquence d'ADN codant pour un polypeptide gag rétroviral modifié formant une particule, dans lequel ledit polypeptide gag contient au moins une séquence d'aminoacides représentant un déterminant antigénique polypeptidique non gag, dans lequel ladite séquence d'ADN codant pour un polypeptide gag est issue du rétrovirus VIH-1, dans lequel ladite séquence d'ADN codant pour ladite séquence d'aminoacides remplace une (des) région(s) de ladite séquence d'ADN codant pour ledit polypeptide gag, ladite (lesdites) région(s) étant capable(s) de présenter au système immunitaire la séquence d'aminoacides codée par la séquence d'ADN insérée, de manière à déclencher une réponse immunitaire, et dans lequel ladite région est localisée aux positions d'aminoacides 99 à 154, 211 à 241 ou 436 à 471, ou à une combinaison quelconque de celles-ci, ledit procédé comprenant le remplacement d'au moins un codon d'une séquence d'ADN codant pour ledit polypeptide gag par une séquence d'ADN codant pour une séquence d'aminoacides non gag.

2. Procédé selon la revendication 1, dans lequel la séquence d'ADN codant pour la séquence d'aminoacides supplémentaire est insérée par l'intermédiaire d'un lieur.

3. Procédé selon la revendication 1 ou 2, dans lequel le déterminant antigénique polypeptidique non-gag est :
(a) au moins une partie du domaine de liaison à CD4 de gp 120 ;
(b) au moins une partie de la région variable 3 de gp 120 ; ou
(c)au moins une partie de la région fusiogène de gp 41.

4. Procédé pour la production d'un vecteur recombinant, comprenant l'insertion d'une séquence d'ADN selon l'une quelconque des revendications 1 à 3, dans une molécule vectrice.

5. Procédé pour la production d'un virus recombinant, comprenant les étapes d'insertion d'une séquence d'ADN selon l'une quelconque des revendications 1 à 3, dans une molécule d'ADN viral.

6. Procédé selon la revendication 5, dans lequel le virus recombinant est un virus de la vaccine.

7. Procédé pour la production d'un hôte portant un vecteur recombinant susceptible d'être obtenu par un procédé selon la revendication 4 ou un virus recombinant susceptible d'être obtenu par un procédé selon la revendication 5 ou 6, ledit procédé comprenant l'incorporation dudit vecteur recombinant ou dudit virus recombinant dans un hôte approprié.

8. Procédé pour la production d'un polypeptide gag rétroviral modifié qui est codé par une séquence d'ADN selon l'une quelconque des revendications 1 à 3, comprenant les étapes de culture d'un hôte susceptible d'être obtenu par un procédé selon la revendication 7, dans des conditions appropriées, et la récupération dudit polypeptide à partir du milieu.

9. Procédé selon la revendication 8, dans lequel les polypeptides forment des particules.

10. Procédé selon la revendication 8 ou 9, dans lequel ledit organisme hôte est une cellule d'insecte, de préférence une cellule de *Spodoptera frugiperda*, et dans lequel ledit virus par lequel ladite cellule d'insecte est transformée est un virus d'insecte recombinant, de préférence un baculovirus.

11. Procédé selon la revendication 8 ou 9, dans lequel la cellule hôte est une cellule d'insecte, de préférence une cellule Schneider de *Drosophila,* et dans lequel ledit vecteur recombinant par lequel ladite cellule d'insecte est transformée est réplicable de façon stable dans des cellules d'insectes.

## Revendications (Revendications pour l'(les) Etat(s) contractant(s) suivant(s): GR)

1. Polypeptide gag rétroviral modifié formant une particule, dans lequel ledit polypeptide gag contient au moins une séquence d'aminoacides représentant un déterminant antigénique polypeptidique non gag, dans lequel le polypeptide gag est p55 de VIH-1, dans lequel ladite séquence d'aminoacides remplace une (des) région(s) dudit polypeptide gag, ladite (lesdites) région(s) étant capable(s) de présenter au système immunitaire la séquence d'aminoacides insérée, de manière à déclencher une réponse immunitaire, et dans lequel ladite région est localisée aux positions d'aminoacides 99 à 154, 211 à 241, ou 436 à 471, ou à une combinaison quelconque de celles-ci.

2. Polypeptide gag rétroviral modifié, selon la revendication 1, dans lequel le déterminant antigénique polypeptidique non-gag est :
(a) au moins une partie du domaine de liaison à CD4 de gp 120 ;
(b) au moins une partie de la région variable 3 de gp 120 ; ou
(c) au moins une partie de la région fusiogène de gp 41.

3. Séquence d'ADN codant pour le polypeptide gag rétroviral modifié, selon la revendication 1 ou 2.

4. Séquence d'ADN selon la revendication 3, dans laquelle la séquence d'ADN codant pour la séquence d'aminoacides est insérée par l'intermédiaire d'un lieur.

5. Vecteur recombinant contenant une séquence d'ADN selon la revendication 3 ou 4.

6. Virus recombinant contenant une séquence d'ADN selon la revendication 3 ou 4.

7. Virus recombinant selon la revendication 6, qui est un virus de la vaccine.

8. Organisme hôte qui est transformé par un vecteur recombinant selon la revendication 5 ou un virus recombinant selon la revendication 6 ou 7.

9. Procédé pour la production d'un polypeptide gag rétroviral modifié, selon la revendication 1 ou 2, comprenant la culture d'un organisme hôte selon la revendication 8, dans des conditions appropriées, et la récupération du produit d'expression à partir du milieu.

10. Procédé selon la revendication 9, dans lequel ledit organisme hôte est une cellule d'insecte, de préférence une cellule de *Spodoptera frugiperda*, et dans lequel ledit virus par lequel ladite cellule d'insecte est transformée est un virus d'insecte recombinant, de préférence un baculovirus.

11. Procédé selon la revendication 9, dans lequel la cellule hôte est une cellule d'insecte, de préférence une cellule Schneider de *Drosophila,* et dans lequel ledit vecteur recombinant par lequel ladite cellule d'insecte est transformée est réplicable de façon stable dans des cellules d'insectes.

12. Utilisation d'un virus recombinant selon la revendication 6 ou 7, ou d'un polypeptide gag rétroviral modifié, selon la revendication 1 ou 2, éventuellement en association avec un véhicule et/ou diluant pharmaceutiquement acceptable, pour la fabrication d'un vaccin.
